(19) **Europäisches Patentamt / European Patent Office / Office européen des brevets**

(11) **EP 2 816 377 B1**

(12) **FASCICULE DE BREVET EUROPEEN**

(45) Date de publication et mention
de la délivrance du brevet:
**27.02.2019 Bulletin 2019/09**

(51) Int Cl.:
***G01V 11/00*** *(2006.01)*

(21) Numéro de dépôt: **14305900.4**

(22) Date de dépôt: **13.06.2014**

(54) **PROCÉDÉ POUR PRÉDIRE LA QUANTITÉ ET LA COMPOSITION DES FLUIDES PRODUITS PAR DES RÉACTIONS MINÉRALES OPÉRANT DANS UN BASSIN SÉDIMENTAIRE**

VERFAHREN ZUR VORHERSAGE DER MENGE UND ZUSAMMENSETZUNG VON FLÜSSIGKEITEN, DIE DURCH MINERALISCHE REAKTIONEN IN EINEM SEDIMENTBECKEN ERZEUGT WERDEN

METHOD FOR PREDICTING THE AMOUNT AND COMPOSITION OF FLUIDS PRODUCED BY MINERAL REACTIONS OPERATING IN A SEDIMENTARY BASIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.06.2013 FR 1355860**

(43) Date de publication de la demande:
**24.12.2014 Bulletin 2014/52**

(73) Titulaires:
• **IFP Energies nouvelles**
**92500 Rueil-Malmaison (FR)**
• **Total S.A.**
**92400 Courbevoie (FR)**

(72) Inventeurs:
• **BROSSE, Etienne**
**78160 Marly-le-Roi (FR)**

• **RUDKIEWICZ, Jean-Luc**
**92160 ANTONY (FR)**
• **PARRA, Teddy**
**75018 PARIS (FR)**
• **GUICHET, Xavier**
**92500 Rueil-Malamaison (FR)**

(74) Mandataire: **IFP Energies nouvelles**
**Rond-point de l'échangeur de Solaize**
**BP3**
**69230 Solaize (FR)**

(56) Documents cités:
**EP-A1- 1 884 620     EP-A1- 2 110 686**
**FR-A1- 2 830 646**

## Description

La présente invention concerne le domaine de l'industrie pétrolière, et plus particulièrement le domaine de l'exploration des hydrocarbures dans les bassins sédimentaires.

En particulier, l'invention concerne un procédé permettant de calculer la quantité et la composition des fluides d'origine minérale générés par les réactions qui surviennent entre les minéraux des roches sédimentaires à mesure qu'elles s'enfouissent au cours de l'histoire géologique d'un bassin. Ces fluides, caractérisés par des teneurs élevées en eau ($H_2O$) et/ou en dioxyde de carbone ($CO_2$), se forment typiquement au-dessus d'une température (T) de 250°C et d'une pression de pore (P) de 100 MPa, conditions qui sont atteintes dans les parties profondes de certains bassins.

Un enjeu majeur en exploration pétrolière et gazière est de repousser au maximum l'épuisement des ressources en hydrocarbures. Dans une telle perspective, des zones peu explorées compte tenu des coûts de forage associés aux conditions de température (T) et pression (P) élevées commencent à être considérées comme potentiellement économiques. Mais les hydrocarbures que l'on y rencontre, dominés par le méthane ($CH_4$) d'origine "thermogénique" (craquage de la matière organique), sont souvent mélangés à des gaz non hydrocarbures, typiquement $CO_2$ et/ou $N_2$, dans des proportions telles, que les gisements découverts se révèlent commercialement inexploitables.

Ainsi, être capable d'évaluer, avant le forage d'une zone d'un bassin sédimentaire, si dans celle-ci la composition des fluides accumulés dans les pièges peut avoir été fortement influencée par l'apport de fluides non hydrocarbures représente un intérêt considérable en exploration.

### Présentation de l'art antérieur

Une approche visant à prédire le niveau d'un constituant volatil en fonction de la position dans le bassin sédimentaire, à partir de réactions minérales, a été présentée spécifiquement pour le $CO_2$ (L.M. Cathles & M. Schoell, 2007, "Modeling CO2 generation, migration, and titration in sedimentary basins", Geofluids Vol.7, pp.441-450). Elle consiste à calculer la pression partielle de $CO_2$, $pCO_2$ (ou sa fugacité, $fCO_2$), à partir d'un ou plusieurs "tampons minéraux", c'est-à-dire d'un ou plusieurs assemblages de minéraux considérés comme présents dans le bassin, et en état d'équilibre. Ce faisant, il n'est pas possible d'évaluer le comportement d'une lithologie particulière. L'approche est au contraire globale, pour le bassin pris dans son ensemble. L'étude de tout nouveau bassin exige de pouvoir estimer, ou calibrer, la "capacité" de chaque tampon minéral retenu, c'est-à-dire l'importance quantitative qui est en moyenne le sien dans les zones du bassin concerné qui sont productrices de $CO_2$.

À l'inverse, le procédé selon l'invention permet de calculer pas-à-pas le bilan de toutes les réactions minérales susceptibles de produire des constituants volatils au cours de l'histoire géologique d'un bassin, sous la seule contrainte de savoir attribuer des compositions aux roches considérées comme sources, et d'avoir accès au chemin (T,P) en fonction du temps.

Ainsi, l'objet de l'invention concerne un procédé pour prédire une quantité et une composition de fluides d'origine minérale générés dans un bassin sédimentaire par des réactions survenant entre des minéraux de roches sédimentaires à mesure que lesdites roches s'enfouissent au cours de l'histoire géologique dudit bassin.

L'invention fournit donc un outil pour évaluer la quantité de fluides pour l'essentiel non hydrocarbures ($H_2O$, $CO_2$, $N_2$, etc.) générés dans le bassin, et la part de ces fluides qui a pu ensuite migrer jusque dans des roches réservoirs, pour se mélanger aux hydrocarbures d'origine organique, notamment gazeux.

Elle représente une avancée très significative pour les géologues qui recherchent de nouvelles sources d'énergie fossile, en particulier dans les roches réservoirs profondes qui peuvent contenir d'abondantes ressources en gaz naturel.

### La méthode selon l'invention

L'invention concerne un procédé mis en oeuvre par ordinateur pour prédire une quantité et une composition de fluides d'origine minérale générés dans un bassin sédimentaire par des réactions survenant entre des minéraux de roches sédimentaires à mesure que lesdites roches s'enfouissent au cours de l'histoire géologique dudit bassin, dans lequel :

    i. on acquiert des données géologiques caractéristiques dudit bassin ;
    ii. on construit une représentation dudit bassin par un maillage ;
    iii. on calcule, pour au moins un ensemble de mailles dudit maillage, et au moyen d'un modèle de bassin et desdites données géologiques, l'évolution des paramètres suivants : une profondeur d'enfouissement (z), une température (T), une pression de pore (P), un volume (V) et une porosité ($\Phi$) à des âges successifs $t_i$ représentatifs de l'histoire géologique du bassin ;
    iv. on détermine en chaque maille dudit ensemble de mailles, une composition minéralogique ou chimique de roche à partir desdites données géologiques du bassin ;
    v. on détermine la quantité et la composition de fluides d'origine minérale au sein dudit ensemble de mailles, au moyen d'un modèle géochimique et à partir desdits paramètres et de ladite composition et d'une base de donnée thermodynamique.

Selon l'invention, après l'étape v. on peut déterminer une quantité desdits fluides ayant migré jusque dans des roches réservoirs, en déterminant des propriétés thermodynamiques desdits fluides, telles que la répartition en différentes phases fluides ou solides et/ou les densités et viscosités desdites phases, au moyen d'une équation d'état, et au moyen d'un modèle de migration en fonction desdites propriétés de fluides.

L'ensemble de mailles peut correspondre à des mailles sources, une maille source étant une maille dans laquelle la composition de la roche, la pression et la température sont propices à la formation de fluides d'origine minérale.

La température peut être supérieure à 250 °C et la pression est supérieure à 100 MPa.

A l'étape v, on peut réaliser les étapes suivantes :

- on détermine une suite de réactions qui, le long d'un chemin Température-Pression donné, fait passer une roche de composition chimique donnée par une succession de compositions minérales stables ;

- on détermine des variations de quantités de minéraux, ainsi que des quantités de constituants du fluide échangées, au cours de ladite suite de réactions.

On peut déterminer des variations de quantités de minéraux, ainsi que des quantités de constituants du fluide échangées, au cours de cette suite de réactions, au moyen des étapes suivantes :

a. on identifie un système stable pour l'âge $t_{i+1}$ à partir de la composition à un âge $t_i$ ;

b. on identifie des réactions minérales qui font passer du système stable pour l'âge $t_i$ au système stable pour l'âge $t_{i+1}$ ;

c. on réalise un calcul du bilan quantitatif, en masse et/ou nombre de moles, des échanges opérés par ces réactions ;

d. on réalise un calcul du bilan quantitatif, en volume, de ces mêmes échanges :

i. à partir d'une base de données thermodynamiques pour lesdits minéraux ;
ii. à partir d'une l'équation d'état permettant de calculer la composition et la densité de chacune des phases du fluide ;

e. on compare des variations de volume obtenues respectivement par modélisation géochimique $\delta_{i+1}$ et par modélisation de bassin $\Delta_{i+1}$ : si $\delta_{i+1}$ dépasse $\Delta_{i+1}$ d'une quantité jugée tolérable au regard de la précision attendue des bilans de fluide dans le modèle de bassin, telle que par exemple 1 % en valeur relative, on modifie la composition du système en lui

ôtant un volume $\delta_{i+1}$ - $\Delta_{i+1}$ de fluide, soit selon la composition du fluide total, soit selon celle de la phase la moins dense, soit encore selon celle d'un mélange de chacune des phases dans des proportions décidées en fonction des valeurs prises par une propriété calculée pour le fluide telle que la viscosité ;

f. on met en mémoire la quantité et la composition du fluide soustrait du système pour fixer la nouvelle composition qu'il prend à l'âge $t_{i+1}$ ;

g. la nouvelle composition du système devient celle prise en compte pour la modélisation géochimique lors du passage à l'âge suivant ($t_{i+1} \rightarrow t_{i+2}$).

La variation de volume obtenue par modélisation géochimique peut s'écrire :

$\delta_{i+1} = \{(V_m)_{i+1} + (V_f)_{i+1}\} - \{(V_m)_i + (V_f)_i\}$, où $(V_f)_i$ et $(V_m)_i$ représentent respectivement le volume de fluide et le volume de minéraux pour l'âge $t_i$ ;

et la variation de volume obtenue par modélisation de bassin s'écrit :
$\Delta_{i+1} = V_{i+1} - V_i$, où $V_i$ représente le volume de maille obtenue par modélisation.

Les fluides peuvent être caractérisés par des teneurs élevées en eau et/ou en dioxyde de carbone et/ou en gaz hydrocarbures et/ou en hydrogène et/ou en azote et/ou en hydrogène sulfuré.

**Présentation sommaire des figures**

- La figure 1 illustre la séquence opératoire pour la mise en oeuvre du procédé selon l'invention.

- La figure 2 est une coupe verticale dans le modèle de bassin pris pour exemple, localisation d'une maille-source (MS) et d'un niveau réservoir (NR), trajet T-P-z-Φ reconstitué par le modèle pour la maille-source.

- La figure 3 illustre une évolution du système géochimique pris pour exemple : évolution en système fermé, permettant d'illustrer les réactions de genèse de $CO_2$ et de $H_2O$ (dans cet exemple), et les variations de volume associées.

- La figure 4 illustre une évolution du système géochimique pris pour exemple : évolution de la maille-source entre les deux premières étapes de modélisation de bassin où l'ampleur des réactions minérales oblige à prendre en compte un système ouvert (22 et 25 Ma), et mise en évidence de l'effet produit par soustraction d'un "paquet" de fluide.

- La figure 5 représente des cartes des quantités cumulées de constituants expulsés ($H_2O$ et $CO_2$, dans l'exemple traité), aux deux âges illustrés (22 et 25 Ma) (en $kg \cdot m^{-2}$ d'unité géologique source).

- La figure 6 représente des blocs-diagrammes (à 22 et 25 Ma) illustrant le piégeage des constituants expulsés vers le niveau réservoir, *via* une procédure de migration par *ray-tracing*, et leur effet cumulatif, dans les pièges, à des hydrocarbures produits par ailleurs.

**Description détaillée du procédé**

La prédiction quantitative des fluides non hydrocarbures générés dans les bassins est possible à condition de résoudre une série de questions techniques.

Tout d'abord, il faut identifier, pour une composition donnée de roche, quelles réactions sont susceptibles de délivrer de tels fluides, dans quelles conditions de T et P elles se produisent, et quel est leur bilan.

Les minéraux constitutifs des roches sédimentaires sont hérités d'une suite de processus :

- l'altération continentale, qui produit en particulier des quantités importantes de minéraux argileux plus ou moins hydratés ;

- le transport jusqu'aux milieux de dépôt, qui s'accompagne de tris granulométriques mais aussi de mélanges, notamment avec des minéraux carbonatés provenant de divers organismes marins ;

- les modifications postérieures au dépôt, dites "diagénétiques", qui permettent à de nouveaux minéraux de précipiter au sein de la porosité, ou bien en remplacement d'autres minéraux qui se dissolvent.

De tous ces processus, souvent longs et complexes, résulte le spectre assez large des compositions minérales observées, par exemple dans les carottes récupérées en forage. Tant que les sédiments n'ont subi qu'un enfouissement (z) modéré, qui se traduit par des valeurs encore modestes de température (T) et pression (P) (typiquement, T inférieure à 100 ou 150°C, P inférieure à 20 ou 30 MPa), les minéraux détritiques dont la composition est riche en eau (typiquement des argiles, par exemple des smectites à deux couches d'eau interfoliaires) se maintiennent stables. Ensuite, et de manière de plus en plus marquée à mesure que les paramètres z, T et P augmentent, les assemblages minéraux ont tendance à se transformer en de nouveaux assemblages plus pauvres en $H_2O$ (ou en groupements - OH). Il en va de même pour d'autres groupements d'atomes présents dans la structure cristalline des minéraux, et capables de se transformer en constituants volatils (comme $CO_2$ ou $N_2$) dès lors que des réactions peuvent faire passer d'un assemblage minéral à un autre plus stable.

Les réactions évoquées sont des processus géochimiques connus en sciences de la Terre, pour décrire la "dévolatilisation" dans le domaine du "métamorphisme" (P. Eskola, 1921, "The minerai facies of rocks", Norsk Geologisk Tidsskrift Vol.6, pp.142-194 ; N.L. Bowen, 1940, "Prograde metamorphism of siliceous limestone and dolomite, Journal of Geology Vol.48, pp.225-274).

Dans certains bassins, où le gradient géothermique et/ou l'épaisseur des dépôts sont grands, les conditions de température et pression atteintes dans les sédiments les plus enfouis sont telles (plus de 400°C, plus de 200 MPa) que des quantités importantes de constituants volatils peuvent être formées par ces réactions. La nature des assemblages minéraux en jeu, les réactions qui font passer de l'un à l'autre, et les conditions dans lesquelles ces réactions se produisent, peuvent être prédites à partir :

(1) d'une description suffisamment détaillée des propriétés thermodynamiques des minéraux existants, accessible aujourd'hui dans des bases de données dédiées (dont certaines sont publiées) ;

(2) d'un code informatique permettant, à partir de ces propriétés thermodynamiques et d'une composition de roche, de calculer quel est en toutes conditions l'assemblage le plus stable, et quelles quantités de matière sont échangées dans les réactions successives qui font progresser le système vers l'état le plus stable. On appelle "outil de modélisation géochimique" ce type de code. Il en existe de multiples, destinés à l'étude du métamorphisme.

La seconde question à résoudre concerne les variations de volume induites par les réactions. Les molécules libérées par les évolutions minérales ci-dessus ($H_2O$, $CO_2$, $N_2$, *etc.*) sont à l'état de fluide dans les conditions examinées. Les réactions modifient la composition minérale, celle du fluide, et par voie de conséquence les volumes de matière minérale et de fluide. Très généralement, le volume du fluide augmente. Comme la variation de volume de la roche (minéraux + fluide) est contrainte par l'évolution de la géométrie du bassin et par celle de la "compaction" (ou tassement) des sédiments, deux paramètres décrits par un outil habituel de modélisation de bassin tel que le logiciel TemisFlow® (IFP Energies nouvelles, France), il est possible de calculer simplement, en fonction du progrès des réactions et dans l'hypothèse où l'influence des réactions sur la pression n'est pas prise en compte, la part du fluide qui peut rester en place dans le volume poreux, et la part qui devrait en être expulsée.

Une question complémentaire a trait à la nature et au comportement du fluide. Selon sa composition, et les valeurs de T et P, le mélange fluide est constitué d'une ou plusieurs phases. Dans le calcul d'une part expulsée du fluide, tel que présenté ci-dessus, il peut être souhaitable de traiter de manière différente chacune des phases, en fonction de la valeur prise par certaines de leurs caractéristiques respectives (densité, viscosité). La nature et le comportement du fluide peuvent être prédits par la conjonction (1) d'une "équation d'état" (souvent, une équation cubique reliant pression, volume et température, et intégrée dans un code informatique), capable de calculer l'état des phases du fluide, leur composition et leur volume, donc leur densité ; (2) de modèles paramétrés capables de calculer d'autres grandeurs éventuellement requises, par exemple la viscosité.

La dernière question est relative à la migration du fluide expulsé. Dans la perspective du procédé qui fait l'objet de cette présentation, la migration est considérée à l'aide d'une procédure de *ray-tracing* déjà incluse dans la modélisation de bassin.

Le procédé selon l'invention consiste à combiner, selon une suite d'étapes cohérentes, les solutions qui peuvent être apportées à chacune de ces questions techniques.

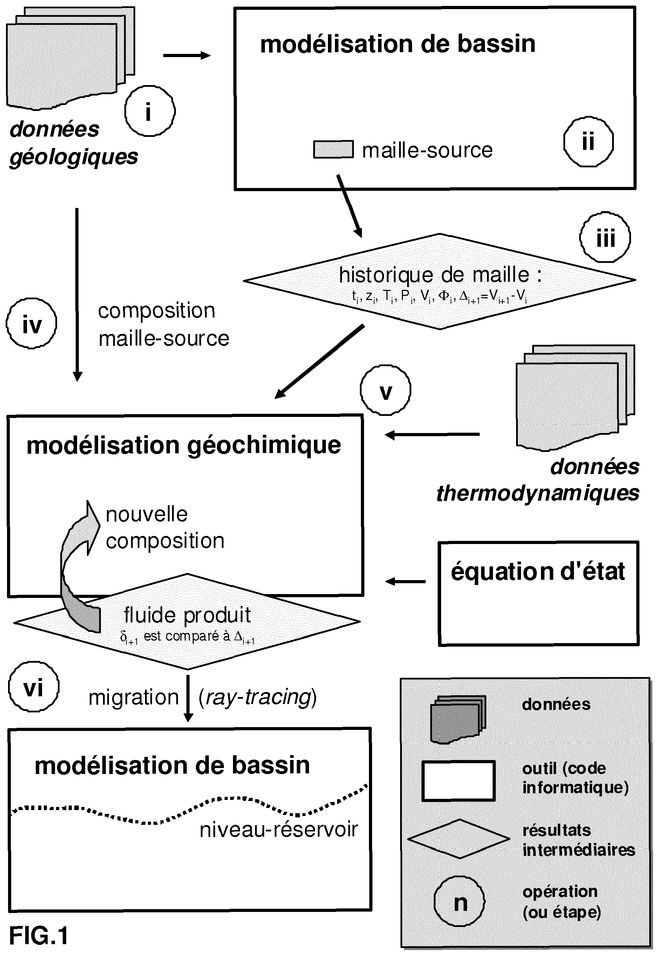

La figure 1 illustre les étapes du procédé selon l'invention pour prédire la quantité et la composition de fluides d'origine minérale générés dans un bassin sédimentaire. Ce procédé comporte les étapes suivantes :

i. Acquisition de données géologiques caractéristiques du bassin sédimentaire

ii. Construction d'une représentation du bassin par un maillage

iii. Calcul de paramètres physiques (z, T, P, V, $\Phi$) pour différents âges

iv. Détermination de la composition minéralogique ou chimique des roches sources

v. Détermination de la quantité et de la composition des fluides d'origine minérale, dans les roches sources
Et une étape optionnelle :

vi. Migration des fluides d'origine minérale dans les réservoirs

Selon l'invention, un fluide d'origine minérale est caractérisé par des teneurs élevées en eau et/ou en dioxyde de carbone et/ou en gaz hydrocarbures et/ou en hydrogène et/ou en azote et/ou en hydrogène sulfuré.

i. Acquisition de données géologiques caractéristiques du bassin sédimentaire

Les données géologiques requises pour la modélisation de bassin sont en partie décrites dans le document suivant :

- J.L. Rudkiewicz et al., 2000, "Integrated basin modeling helps to decipher petroleum systems"; R. Melo and B.J. Katz, eds, "Petroleum systems of South Atlantic Margin", AAPG Memoir Vol. 73, pp.27-40.).

Il s'agit au moins des données suivantes :

- géométrie des couches à l'époque actuelle en une (z), deux (x,z) ou trois (x,y,z) dimensions,

- nature des roches composant les couches,

- âge géologique des couches,

- températures, pressions, porosités observées dans les forages,

- si nécessaire, épaisseurs sédimentaires érodées et âges d'érosion.

Ces données sont acquises par des moyens connus des spécialistes (diagraphies, carottes, ...).

ii. Construction d'une représentation du bassin par un maillage

La construction d'une représentation d'un bassin sédimentaire est une étape connue des spécialistes. Le maillage peut être en une (z), deux (x,z) ou trois (x,y,z) dimensions.

iii. Calcul de paramètres physiques (z, T, P, V, $\Phi$) pour différents âges

Au cours de cette étape, on détermine l'évolution des paramètres suivants pour un ensemble de mailles du maillage : une profondeur d'enfouissement (z), une température (T), une pression de pore (P), un volume (V) et une porosité ($\Phi$) à des âges successifs ($t_i$) représentatifs de l'histoire géologique du bassin.

Pour ce faire, on utilise un outil de modélisation de bassin, appelé "modèle de bassin", et les données géologiques acquises à l'étape i. Un modèle de bassin est un logiciel dédié à la modélisation de bassin sédimentaire, capable de calculer, en toute maille et pour tout temps géologique (t), les valeurs atteintes par la profondeur d'enfouissement (z), la température (T), la pression de pore (P), le volume (V) et la porosité ($\Phi$). Remarque : V(z) et $\Phi$(z) définissent ce qui est appelé "compaction". TemisFlow® (IFP Energies nouvelles, France) est un exemple de modèle de bassin.

A partir de ce logiciel, utilisé sur un ordinateur, on détermine donc des "*historiques de maille*", c'est-à-dire des tableaux {$t_i$, $z_i$, $T_i$, $P_i$, $V_i$, $\Phi_i$} contenant la valeur de z, T, P, V et $\Phi$ d'une maille à des moments successifs $t_i$ représentatifs de l'histoire géologique du bassin (épisodes de dépôt ou d'érosion des couches sédimentaires).

On peut limiter la reconstitution de ces historiques aux seules mailles qui sont considérées comme des sources potentielles de fluides d'origine minérale. Ces mailles sont appelées "mailles-sources". Une maille-source peut être définie comme une maille dans laquelle la composition de la roche, la pression et la température sont propices à la formation de fluides d'origine minérale, la pression et la température ayant atteint un certain seuil au cours de leur évolution géologique (par exemple, une température supérieure à 250 °C et une pression supérieure à 100 MPa).

iv. Détermination de la composition minéralogique ou chimique des roches sources

Au cours de cette étape, on détermine pour chaque maille-source la composition minéralogique (ou chimique) représentative, c'est-à-dire un ensemble de minéraux susceptibles de décrire d'une manière jugée comme suffisamment représentative et complète la composition des roches du bassin dans cette maille.

Cette composition minéralogique (ou chimique) est inférée des données géologiques du bassin acquises à l'étape initiale. Elle peut être déterminée de manière directe par analyse minéralogique des déblais remontés lors du forage d'un ou plusieurs puits, ou par l'examen minéralogique des carottes de roches prélevées lors de tels sondages. Elle peut aussi être déterminée de manière indirecte, par l'analyse des diagraphies électriques enregistrées le long des puits. Une autre méthode consiste à utiliser les mesures géophysiques indirectes (sismique réflexion, magnétométrie ou gravimétrie) qui renseignent sur les grandes classes de composition. Enfin, une dernière méthode consiste à rechercher des ensembles lithologiques qui se présentent comme des équivalents latéraux des couches d'intérêt et qui affleurent à proximité.

v. Détermination de la quantité et de la composition des fluides d'origine minérale, dans les roches sources

Au cours de cette étape, on détermine au sein de l'ensemble de mailles-sources la quantité et la composition des fluides d'origine minérale. Pour ce faire, on utilise :

- un modèle géochimique ;

- les paramètres z, T, P, V et $\Phi$ ;

- la composition minéralogique ou chimique des roches sources ;

- une base de donnée thermodynamique contenant, pour un ensemble de minéraux susceptibles de décrire la composition des roches du bassin, les paramètres thermodynamiques permettant de connaître et / ou de calculer en toutes conditions de température et pression, c'est-à-dire l'enthalpie libre de Gibbs de chaque minéral et le volume molaire de chaque minéral.

Un modèle géochimique (Theriak/Domino par exemple) est un logiciel capable de calculer en toutes conditions de température et pression, et à partir de la base de donnée thermodynamique :

- la composition minérale stable d'une roche de composition chimique donnée ;

- la suite des réactions qui, le long d'un chemin T-P donné, fait passer la roche par une succession de compositions stables ;

- les quantités de minéraux et de constituants du fluide échangées au cours de cette suite de réactions.

Ainsi, cette étape v. comporte les sous-étapes suivantes :

- on détermine une suite de réactions qui, le long d'un chemin Température-Pression donné, fait passer une roche de composition chimique donnée par une succession de compositions minérales stables ;

- on détermine des variations de quantités de minéraux, ainsi que des quantités de constituants du fluide échangées, au cours de ladite suite de réactions.

Pour ce faire, on réalise les itérations suivantes pour chaque maille-source :

a. on identifie un système (assemblage minéral + fluide) stable pour l'âge $t_{i+1}$ (conditions $T_{i+1}$, $P_{i+1}$) à partir de la composition à un âge $t_i$ ;
b. on identifie des réactions minérales qui font passer du système stable pour l'âge $t_i$ au système stable pour l'âge $t_{i+1}$ ;
c. on réalise un calcul du bilan quantitatif, en masse et/ou nombre de moles, des échanges opérés par ces réactions (i.e., minéraux créés ou détruits, constituants du fluide créés ou détruits) ;
d. on réalise un calcul du bilan quantitatif, en volume, de ces mêmes échanges :

- à partir d'une base de données thermodynamiques pour lesdits minéraux ;
- à partir d'une l'équation d'état permettant de calculer la composition et la densité de chacune des phases du fluide ; un exemple d'équation d'état est donné dans le document suivant :
- Z. Duan, N. Moller & J.H. Weare, 1992, "An equation of state (EOS) for CH4-CO2-H2O system: I. Pure systems from 0 to 1000 °C and 0 to 8000 bar", Geochimica Cosmochimica Acta Vol.56, pp.2605-2617.

e. on compare des variations de volume obtenues respectivement par modélisation géochimique $\delta_{i+1}$ et par modélisation de bassin (modélisation de la compaction) $\Delta_{i+1}$ : si $\delta_{i+1}$ dépasse $\Delta_{i+1}$ d'une quantité jugée tolérable au regard de la précision attendue des bilans de fluide dans le modèle de bassin, telle que par exemple 1 % en valeur relative, on modifie la composition du système en lui ôtant un volume $\delta_{i+1} - \Delta_{i+1}$ de fluide, soit selon la composition du fluide total, soit selon celle de la phase la moins dense, soit encore selon celle d'un mélange de chacune des phases dans des proportions décidées en fonction des valeurs prises par une propriété calculée pour le fluide telle que la viscosité ;

f. on met en mémoire la quantité et la composition du fluide soustrait du système pour fixer la nouvelle composition qu'il prend à l'âge $t_{i+1}$ ;

g. la nouvelle composition du système devient celle prise en compte pour la modélisation géochimique lors du passage à l'âge suivant ($t_{i+1} \rightarrow t_{i+2}$).

La variation de volume obtenue par modélisation géochimique peut s'écrire, dans la maille-source :

$$\delta_{i+1} = \left\{ \left( V_m \right)_{i+1} + \left( V_f \right)_{i+1} \right\} - \left\{ \left( V_m \right)_i + \left( V_f \right)_i \right\}$$

où $(V_f)_i$ et $(V_m)_i$ représentent respectivement le volume de fluide et le volume de minéraux pour l'âge $t_i$.

La variation de volume obtenue par modélisation de bassin s'écrit :

$$\Delta_{i+1} = V_{i+1} - V_i$$

où $V_i$ représente le volume de la maille-source, obtenu par modélisation de bassin.

vi. Migration des fluides d'origine minérale dans les réservoirs

On détermine la quantité des fluides ayant migré jusque dans des roches réservoirs. Pour ce faire, on détermine les propriétés du fluide (état des phases, composition et densité de chacune des phases) au moyen d'une équation d'état, utilisable dès lors que la température, la pression et la composition globale du fluide sont connus.

Enfin, on utilise ces propriétés au sein d'un modèle de migration. Une procédure de ray-tracing est une des options possibles pour le calcul de la migration. Ce type de procédure est disponible dans l'outil de modélisation de bassin (TemisFlow® (IFP Energies nouvelles, France) par exemple).

Cette procédure est appliquée à l'âge $t_{i+1}$, en considérant que les masses de fluide soustraites aux mailles-sources, obtenues pour l'itération en temps $t_i \rightarrow t_{i+1}$ au cours de l'opération (v) décrite ci-dessus (Détermination de la quantité et de la composition des fluides d'origine minérale, dans les roches sources), sont collectées instantanément dans un "niveau-réservoir" (éventuellement plusieurs), défini(s) au préalable dans les données géologiques.

Le cas échéant, ils y rejoignent des fluides hydrocarbures provenant des "roches-mères" classiquement prises en compte par un modèle de bassin. La géométrie du niveau-réservoir donné, à l'âge $t_{i+1}$, détermine en son sein l'existence de "pièges" pour les fluides.

Par une nouvelle utilisation de l'équation d'état, la composition et la densité de la (ou des) phase(s) fluide(s) sont calculées dans les conditions de température ($T_{i+1}$) et de pression ($P_{i+1}$) de chaque piège à l'âge $t_{i+1}$.

La répartition des fluides dans les pièges est effectuée en fonction de ces propriétés et du volume disponible dans chaque piège. Si plusieurs niveaux-réservoirs sont définis, une règle de répartition des fluides profonds collectés, entre les divers niveaux, est également définie.

**Exemple**

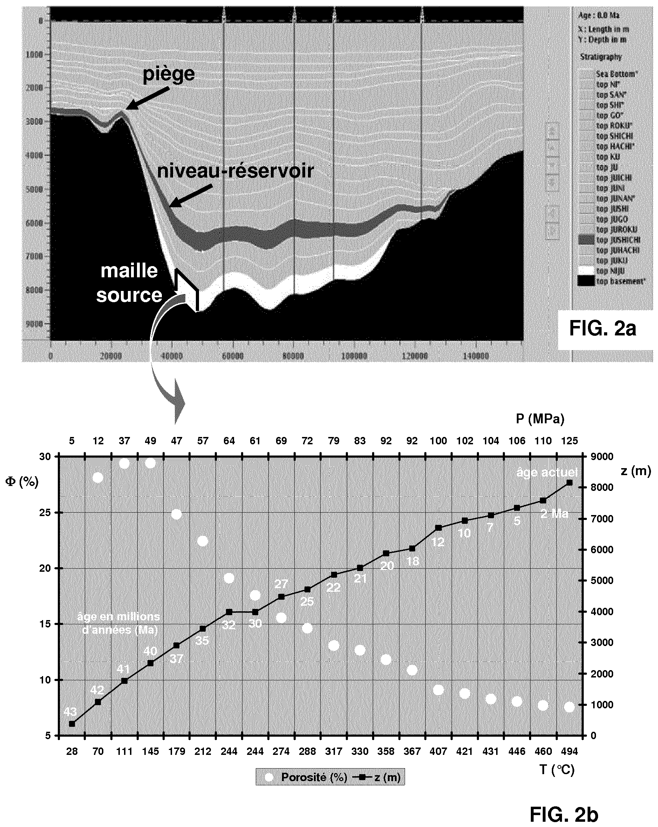

Les figures 2 à 6 illustrent un exemple d'application du procédé selon l'invention. Les caractéristiques géologiques selon cet exemple sont les suivantes :

-   Paramètres liés au bassin sédimentaire
  La figure 2 montre une coupe géologique à travers la représentation de bassin choisie (maillage 3D) et l'histoire d'une maille-source située dans la couche la plus profonde (déposée entre 43 et 42 Ma).
  Pour la maille-source, on utilise le modèle de bassin pour calculer l'évolution de la profondeur (toit de la maille), de la température, de la pression, et de la porosité tout au long des 19 étapes de l'histoire géologique, c'est-à-dire dans les intervalles compris entre les 20 âges de la description temporelle du bassin (43, 42, 41, 40, 37, 35, 32, 30, 27, 25, 22, 21, 20, 18, 12, 10, 7, 5 et 2 Ma avant 0 Ma), comme celle du volume de la maille (non représenté).

-   Composition minéralogique de la maille-source : grès contenant des carbonates diagénétiques, exprimée avec les éléments chimiques Si, Al, Ca, Mg, C, H et O (ni Na, ni K, ni Fe) :

  ∘ proportions en volume, définies à 100°C et 32.5 MPa : 60 % de quartz, 5 % de kaolinite, 15 % de calcite, 10 % de dolomite, porosité Φ de 10 % occupée par un fluide constitué à 90 % de $H_2O$ et 10 % de $CO_2$ ;

  ∘ composition élémentaire correspondante, en nombre de moles pour 1 $dm^3$ de roche (roche = minéraux + fluide présent dans la porosité) : 27,451 mol de Si (silicium), 1,005 mol de Al (aluminium), 5,615 mol de Ca (calcium), 1,554 mol de Mg (magnésium), 7,724 mol de C (carbone), 12 mol de H (hydrogène), 85,026 mol de O (oxygène).

- Niveau-réservoir de collecte des fluides pour la procédure de *ray-tracing* : couche déposée entre 40 et 37 Ma (figure 2).

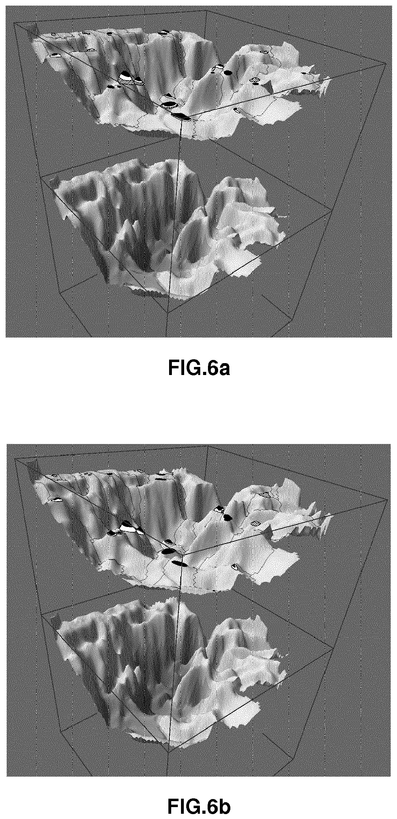

- Structuration du bassin : elle est illustrée par le bloc-diagramme de la figure 6.

Les outils logiciels utilisé dans cet exemple sont les suivants :

1. Logiciel pour la modélisation de bassin (modèle de bassin) : TemisFlow® (IFP Energies nouvelles, France). D'autres modèles de bassin pourraient être utilisés dans le même but.

2. Base de données thermodynamiques : elle est construite sur la base des données issues des références suivantes :

- R.G. Berman : 1988, "Internally-consistent thermodynamic data for minerais in the system Na2O-K2O-CaO-MgO-FeO-Fe2O3-Al2O3-SiO2-TiO2-H2O-CO2", Journal of Petrology Vol.29, pp.485-522

- O. Vidal, T. Parra & F. Trotet, 2001, "A thermodynamic model for Fe-Mg aluminous chlorite using data from phase equilibrium experiments and natural pelitic assemblages in the 100°-600°C, 1-25 kb range", American Journal of Science Vol.301, pp.557-592 ;

- T. Parra, O. Vidal & P. Agard, 2002, "A thermodynamic model for Fe-Mg dioctahedral K-white micas using data from phase equilibrium experiments and natural pelitic assemblages", Contribution to Mineralogy and Petrology Vol.143, pp.706-732 ;

- O. Vidal, T. Parra & P. Vieillard, 2005, "Thermodynamic properties of the Tschermak solid solution in Fe-chlorites: Application to natural examples and possible role of oxidation", American Mineralogist Vol.90, pp.359-370 ;

- O. Vidal, V. De Andrade, E. Lewin, M. Munoz, T. Parra & S. Pascarelli, 2006, "P-T-deformation-Fe3+/Fe2+ mapping at the thin section scale and comparison with XANES mapping. Application to a garnet-bearing metapelite from the Sambagawa metamorphic belt (Japan)", Journal of Metamorphic Geology Vol.24, pp.669-683).

D'autres bases de données pourraient être utilisées dans le même but, par exemple celle de T.J.B. Holland & R. Powell (2011, "An improved and extended internally consistent thermodynamic dataset for phases of petrological interest, involving a new equation of state for solids", Journal of Metamorphic Geology Vol.29, pp.333-383).

3. Code informatique pour la modélisation géochimique (modèle géochimique) : la suite *Theriak/Domino* a été utilisée. Cette suite est décrite dans les références suivantes :

- C. de Capitani & T.H. Brown, 1987, "The computation of chemical equilibrium in complex systems containing non-ideal solutions", Geochimica Cosmochimica Acta Vol.51, pp.2639-2652 ;

- C. de Capitani & K. Petrakakis K., 2010, "The computation of equilibrium assemblage diagrams with Theriak/Domino software", American Mineralogist Vol.95, pp.1006-1016).

D'autres codes informatiques pourraient être utilisés dans le même but, par exemple *Perple_X* (J.A.D. Connolly, 2005, "Computation of phase equilibria by linear programming: A tool for geodynamic modeling and its application to subduction zone decarbonation", Earth & Planetary Science Letters Vol.236, pp.524-541), ou encore *THERMOCALC* (R. Powell, T.J.B. Holland & B. Worley, 1998, "Calculating phase diagrams involving solid solutions via non-linear equations, with examples using THERMOCALC", Journal of Metamorphic Geology Vol.16, pp.577-588).

4. Équation d'état de Z. Duan, N. Moller & J.H. Weare décrite dans :

- 1992, "An equation of state (EOS) for CH4-CO2-H2O system: I. Pure systems from 0 to 1000°C and 0 to 8000 bar", Geochimica Cosmochimica Acta Vol.56, pp.2605-2617 ;

- 1992, "An equation of state (EOS) for CH4-CO2-H2O system: II. Mixtures from 50 to 1000 °C and 0 to 1000 bar", Geochimica Cosmochimica Acta Vol.56, pp.2619-2631).

D'autres équations d'état pourraient être utilisées dans le même but.

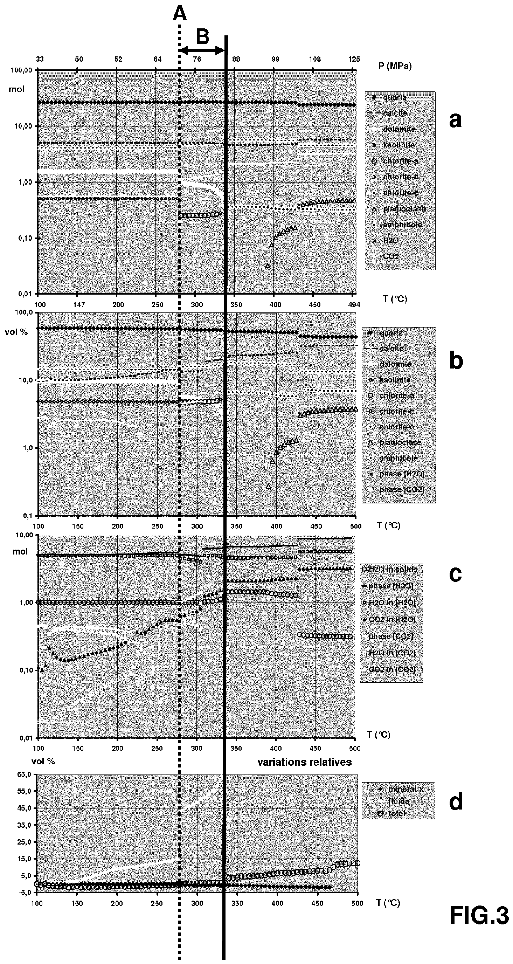

La modélisation géochimique en système fermé, c'est-à-dire sans soustraction de fluide, permet de faire comprendre une évolution-type des compositions et des volumes le long du "chemin $\{t_i, T_i, P_i\}$" (chemin parcouru à T et P croissants), pour le système chimique représentant la maille-source n°1 (figure 3) :

- événement-type A : traduit l'effet d'une réaction uni-variante productrice de fluide et conduisant à une variation brutale des compositions et des volumes (réaction A : dolomite + kaolinite + $H_2O$ → [chlorites] + quartz + calcite + $CO_2$ complète jusqu'à épuisement de la kaolinite) (la notation [chlorites] représente la "solution solide" des chlorites) ;

- épisode-type B : traduit l'effet d'une réaction di-variante au cours de laquelle la solution solide des chlorites change peu à peu de composition (dolomite + quartz + [chlorites 1] + $H_2O$ → [chlorites 2] + calcite + $CO_2$ jusqu'à épuisement de la dolomite) ;

- variation du volume total $\{(V_m)_i + (V_f)_i\}$ (figure 3d) : faible et négligeable avant l'événement A ; positive lors de l'événement A ; positive et progressive durant l'épisode B.

L'événement A survient entre les temps "27 Ma" et "25 Ma" de la modélisation de bassin. Il induit la sortie d'un "paquet" de fluide à l'âge "25 Ma". L'épisode B se déroule progressivement entre les âges "25 Ma" et "22 Ma". Il se poursuit un peu au-delà, mais dès l'âge "22 Ma" on peut tenir compte du fluide qu'il produit pour la migration. Les âges "25 Ma" et "22 Ma" permettent d'illustrer, ici, la procédure d'ouverture du système source et de modification de la composition de la maille-source qui lui est attachée, puis celle de la migration du fluide vers un niveau réservoir sus-jacent à la source.

La mise en oeuvre du procédé peut donc être illustrée de la manière suivante en utilisant cet exemple :

1. Modélisation de bassin : reconstitution de l'historique $\{t_i, z_i, T_i, P_i, V_i, \Phi_i\}$ d'une maille-source (figure 2).

2. Modélisation géochimique et utilisation de l'équation d'état, étape par étape, pour chaque maille-source. Par exemple, entre les temps "43 Ma" et "25 Ma", pour le système chimique représentant la maille-source :

a. cas où, à chaque âge $t_{i+1}$, la variation du volume total $\delta_{i+1}$-$\Delta_{i+1}$ reste dans la tolérance définie pour que le système source demeure fermé (comme à la figure 3 avant l'événement A) : la composition du système n'est pas modifiée pour le calcul géochimique à l'étape suivante ;

b. cas où la variation du volume total $\delta_{i+1}$-$\Delta_{i+1}$ nécessite qu'une partie du fluide soit extraite du système source, comme à l'âge "25 Ma" (figure 3, après l'événement A) et à l'âge "22 Ma" (figure 3, au cours de l'épisode B) :

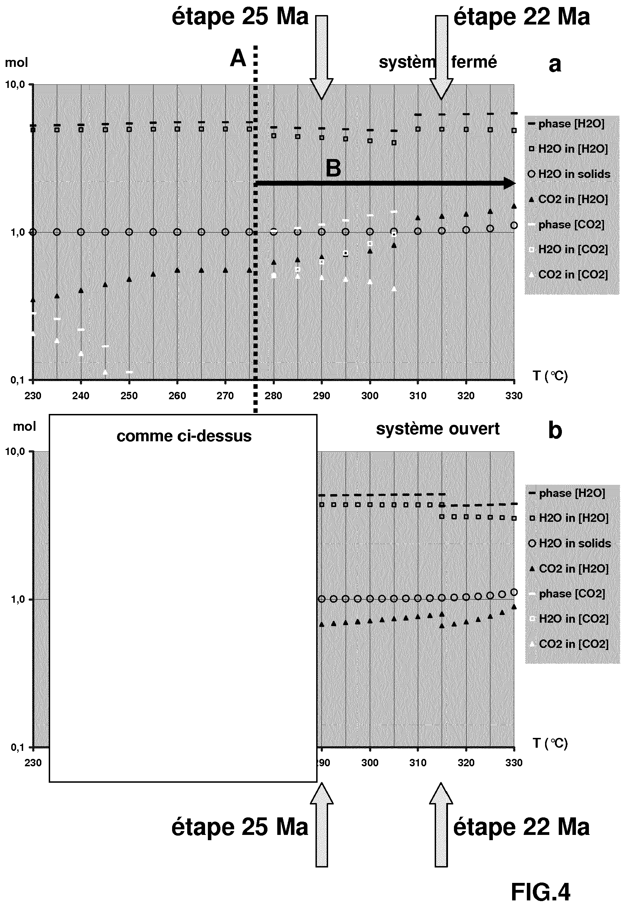

i. l'équation d'état donne la composition de chaque phase fluide à l'âge "25 Ma" (figure 4a) ;

ii. une règle de séparation est appliquée : ici, on choisit de retirer les phases fluides l'une après l'autre, par ordre de densité croissante, ce qui conduit à retirer tout le volume de la "phase $CO_2$" formée (soit environ 5 % du volume total de la roche), ou encore $H_2O$ et $CO_2$ en proportion molaire de leur contribution à cette phase (respectivement 56 % et 44 %) ;

iii. la composition du système est modifiée pour le calcul géochimique entre l'âge "25 Ma" et l'âge "22 Ma" (figure 4b) : il ne se reforme pas de phase $CO_2$, mais à 22 Ma il convient de retirer 17 % de la "phase $H_2O$", qui contient respectivement 85 % de $H_2O$, et 15 % de $CO_2$ en proportion molaire (on poursuivrait alors par un nouveau changement de composition pour la reprise du calcul géochimique, jusqu'à l'âge "21 Ma" -cela n'est pas illustré ici).

3. Modélisation de bassin : pour le calcul de migration par la procédure de *ray-tracing*. À une étape donnée, on procède en collectant les fluides d'un ensemble de mailles-sources (fluides inorganiques, et, éventuellement, hydrocarbures en provenance de roches-mères identifiées par ailleurs). Ici, dans un seul niveau-réservoir.

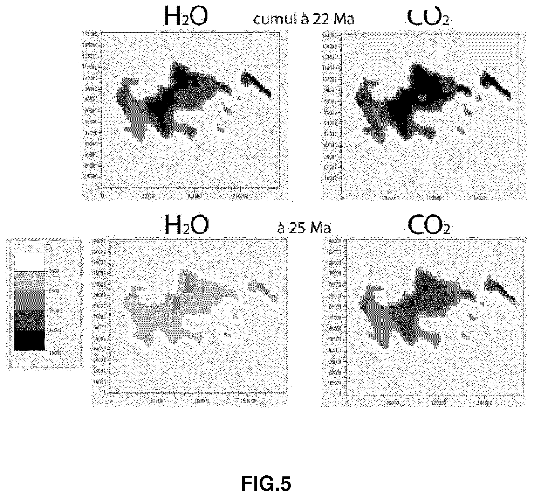

a. La première étape où la migration est illustrée se termine à "25 Ma" : un ensemble de mailles-sources est concerné, pour lequel on montre les quantités de $H_2O$ et de $CO_2$ qui doivent être expulsées (figure 5, en bas).

b. La migration par *ray-tracing,* à 25 Ma, de $H_2O$ et de $CO_2$ produits dans les mailles-sources est combinée à celle de $CH_4$ produit dans des roches-mères : l'application de l'équation d'état au sein de chaque piège structural déterminé par la géométrie du bassin à 25 Ma conduit à la répartition des fluides qui est présentée figure 6a. On y voit une phase riche en $CO_2$, la plus légère, occupant la partie élevée des pièges, et une phase "hydrocarbures" (produits dans des roches-mères par ailleurs), relativement plus dense. L'eau forme la troisième phase et occupe la base de l'espace poreux disponible dans la structure du piège, au sein de la roche réservoir.

c. La seconde étape où la migration est illustrée se termine à "22 Ma" : la figure 5 (en haut) montre, pour cet âge, les quantités cumulées de $H_2O$

et de $CO_2$ expulsées depuis 25 Ma.

d. De la même façon, la migration par *ray-tracing* à 22 Ma conduit à la répartition des fluides qui est présentée figure 6b. Les fluides présents à 25 Ma s'ajoutent à ceux expulsés entre 25 et 22 Ma, et fournissent une nouvelle répartition des phases à 22 Ma. On note que les pièges disponibles à 22 Ma ne sont pas exactement les mêmes que ceux disponibles à 25 Ma.

**Revendications**

1. Procédé mis en oeuvre par ordinateur pour prédire une quantité et une composition de fluides d'origine minérale générés dans un bassin sédimentaire par des réactions survenant entre des minéraux de roches sédimentaires à mesure que lesdites roches s'enfouissent au cours de l'histoire géologique dudit bassin, dans lequel :

   i. on acquiert des données géologiques caractéristiques dudit bassin ;
   ii. on construit une représentation dudit bassin par un maillage ;
   iii. on calcule, pour au moins un ensemble de mailles dudit maillage, et au moyen d'un modèle de bassin et desdites données géologiques, l'évolution des paramètres suivants : une profondeur d'enfouissement (z), une température (T), une pression de pore (P), un volume (V) et une porosité (Φ) à des âges successifs ti représentatifs de l'histoire géologique du bassin ;
   iv. on détermine en chaque maille dudit ensemble de mailles, une composition minéralogique ou chimique de roche à partir desdites données géologiques du bassin ;
   v. on détermine une quantité et une composition de fluides d'origine minérale au sein dudit ensemble de mailles, au moyen d'un modèle géochimique et à partir desdits paramètres et de ladite composition minéralogique ou chimique de roche et d'une base de donnée thermodynamique.

2. Procédé selon la revendication 1, dans lequel après l'étape v. on détermine une quantité desdits fluides ayant migré jusque dans des roches réservoirs, en déterminant des propriétés thermodynamiques desdits fluides, telles que la répartition en différentes phases fluides ou solides et/ou les densités et viscosités desdites phases, au moyen d'une équation d'état, et au moyen d'un modèle de migration en fonction desdites propriétés de fluides.

3. Procédé selon l'une des revendications précédentes, dans lequel ledit ensemble de mailles correspond à des mailles sources, une maille source étant une maille dans laquelle la composition de la roche, la pression et la température sont propices à la formation de fluides d'origine minérale.

4. Procédé selon la revendication 3, dans lequel la température est supérieure à 250 °C et la pression est supérieure à 100 MPa.

5. Procédé selon l'une des revendications précédentes, dans lequel à l'étape v. on réalise les étapes suivantes :

   - on détermine une suite de réactions qui, le long d'un chemin Température-Pression donné, fait passer une roche de composition chimique donnée par une succession de compositions minérales stables ;
   - on détermine des variations de quantités de minéraux, ainsi que des quantités de constituants du fluide échangées, au cours de ladite suite de réactions.

6. Procédé selon la revendication 5, dans lequel on détermine des variations de quantités de minéraux, ainsi que des quantités de constituants du fluide échangées, au cours de cette suite de réactions, au moyen des étapes suivantes :

   a. on identifie un système stable pour l'âge $t_{i+1}$ à partir de la composition à un âge $t_i$ ;
   b. on identifie des réactions minérales qui font passer du système stable pour l'âge $t_i$ au système stable pour l'âge $t_{i+1}$ ;
   c. on réalise un calcul du bilan quantitatif, en masse et/ou nombre de moles, des échanges opérés par ces réactions ;
   d. on réalise un calcul du bilan quantitatif, en volume, de ces mêmes échanges :

      iii. à partir d'une base de données thermodynamiques pour lesdits minéraux ;
      iv. à partir d'une l'équation d'état permettant de calculer la composition et la densité de chacune des phases du fluide ;

   e. on compare des variations de volume obtenues respectivement par modélisation géochimique $\delta_{i+1}$ et par modélisation de bassin $\Delta_{i+1}$ : si $\delta_{i+1}$ dépasse $\Delta_{i+1}$ d'une quantité jugée tolérable au regard de la précision attendue des bilans de fluide dans le modèle de bassin, telle que par exemple 1 % en valeur relative, on modifie la composition du système en lui ôtant un volume $\delta_{i+1} - \Delta_{i+1}$ de fluide, soit selon la composition du fluide total, soit selon celle de la phase la moins dense, soit encore selon celle d'un mélange de chacune des phases dans des proportions décidées en fonction des valeurs prises par une propriété calculée pour le fluide telle que la viscosité ;

f. on met en mémoire la quantité et la composition du fluide soustrait du système pour fixer la nouvelle composition qu'il prend à l'âge $t_{i+1}$ ;

g. la nouvelle composition du système devient celle prise en compte pour la modélisation géochimique lors du passage à l'âge suivant ($t_{i+1} \rightarrow t_{i+2}$).

7. Procédé selon la revendication 6, dans lequel la variation de volume obtenue par modélisation géochimique s'écrit :

$$\delta_{i+1}=\{(V_m)_{i+1}+(V_f)_{i+1}\}-\{(V_m)_i+(V_f)_i\},$$ où $(V_f)_i$ et $(V_m)_i$ représentent respectivement le volume de fluide et le volume de minéraux pour l'âge $t_i$ ;

et la variation de volume obtenue par modélisation de bassin s'écrit :

$$\Delta_{i+1} = V_{i+1}-V_i,$$ où $V_i$ représente le volume de maille obtenue par modélisation.

8. Procédé selon l'une des revendications précédentes, dans lequel les fluides sont **caractérisés par** des teneurs élevées en eau et/ou en dioxyde de carbone et/ou en gaz hydrocarbures et/ou en hydrogène et/ou en azote et/ou en hydrogène sulfuré.

## Patentansprüche

1. Verfahren, das von einem Computer eingesetzt wird, um eine Menge und eine Zusammensetzung von Fluiden mineralischen Ursprungs vorherzusagen, die in einem Sedimentbecken durch Reaktionen erzeugt werden, die zwischen Mineralien von Sedimentgestein in dem Maße auftreten, als sich das Gestein während der geologischen Geschichte des Beckens ablagert, bei dem:

   i. charakteristische geologische Daten des Beckens erfasst werden;
   ii. eine Darstellung des Beckens durch eine Rastergitterbildung erstellt wird,
   iii. für mindestens eine Gesamtheit von Zellen des Rastergitters und mit Hilfe eines Modells eines Beckens und der geologischen Daten die Entwicklung der folgenden Parameter berechnet wird: eine Ablagerungstiefe (z), eine Temperatur (T), ein Porendruck (P), ein Volumen (V) und eine Porosität ($\Phi$) zu aufeinanderfolgenden Zeitaltern $t_i$, die für die geologische Geschichte des Beckens repräsentativ sind;
   iv. an jeder Zelle der Gesamtheit von Zellen eine mineralogische oder chemische Gesteinszusammensetzung auf Basis der geologischen Daten des Beckens bestimmt wird;

   v. eine Menge und eine Zusammensetzung von Fluiden mineralischen Ursprungs innerhalb der Gesamtheit von Zellen mit Hilfe eines geochemischen Modells und auf Basis der Parameter und der mineralogischen oder chemischen Gesteinszusammensetzung und einer thermodynamischen Datenbasis bestimmt wird.

2. Verfahren nach Anspruch 1, bei dem nach dem Schritt v. eine Menge der Fluide bestimmt wird, die bis in Reservoirgestein migriert sind, wobei thermodynamische Eigenschaften der Fluide, wie die Verteilung in verschiedene fluide oder feste Phasen und/oder die Dichten und Viskositäten der Phasen, mit Hilfe einer Zustandsgleichung und mit Hilfe eines Migrationsmodells in Abhängigkeit von den Fluideigenschaften bestimmt werden.

3. Verfahren nach einem der vorhergehenden Ansprüche, bei dem die Gesamtheit der Zellen Quellenzellen entspricht, wobei eine Quellenzelle eine Zelle ist, in der die Zusammensetzung des Gesteins, der Druck und die Temperatur für die Bildung von Fluiden mineralischen Ursprungs günstig sind.

4. Verfahren nach Anspruch 3, bei dem die Temperatur höher als 250 °C und der Druck größer als 100 MPa ist.

5. Verfahren nach einem der vorhergehenden Ansprüche, bei dem in Schritt v. die folgenden Schritte ausgeführt werden:

   - Bestimmung einer Reaktionsfolge, die entlang eines gegebenen Temperatur-Druck-Weges ein Gestein einer gegebenen chemischen Zusammensetzung durch eine Aufeinanderfolge von stabilen mineralischen Zusammensetzungen verlaufen lässt;
   - Bestimmung der Variationen von Mengen von Mineralien sowie Mengen von Bestandteilen des Fluids, die während der Reaktionsfolge ausgetauscht werden.

6. Verfahren nach Anspruch 5, bei dem Variationen von Mengen von Mineralien sowie Mengen von Bestandteilen, die während dieser Reaktionsfolge ausgetauscht werden, mit Hilfe der folgenden Schritte bestimmt werden:

   a. Identifikation eines stabilen Systems für das Zeitalter $t_{i+1}$ auf Basis der Zusammensetzung zu einem Zeitalter $t_i$;
   b. Identifikation der mineralischen Reaktionen, die von dem stabilen System für das Zeitalter $t_i$ zum dem stabilen System für das Zeitalter $t_{i+1}$ übergehen lassen;
   c. Durchführen einer Berechnung der quantitativen Bilanz in Masse und/oder Molzahl der durch diese Reaktionen durchgeführten Austausche;

d. Durchführen einer Berechnung der quantitativen Bilanz in Volumen dieser selben Austausche:

iii. auf Basis einer thermodynamischen Datenbasis für diese Mineralien;
iv. auf Basis einer Zustandsgleichung, die es ermöglicht, die Zusammensetzung und die Dichte jeder der Phasen des Fluids zu berechnen;

e. Vergleich der Volumenvariationen, die durch geochemische Modellierung $\delta_{i+1}$ und durch Modellierung eines Beckens $\Delta_{i+1}$ erhalten werden: wenn $\delta_{i+1}$ um eine für tolerierbar beurteilte Menge $\Delta_{i+1}$ gegenüber der erwarteten Präzision der Fluidbilanzen in dem Beckenmodell überschreitet, wie beispielsweise 1 % in relativem Wert, wird die Zusammensetzung des Systems verändert, wobei ihm ein Volumen $\delta_{i+1} - \Delta_{i+1}$ an Fluid weggenommen wird, entweder gemäß der Zusammensetzung des Gesamtfluids, oder gemäß jener der am wenigsten dichten Phase, oder auch gemäß jener einer Mischung jeder der Phasen in Verhältnissen, die in Abhängigkeit von den Werten beschlossen werden, die durch eine für das Fluid berechnete Eigenschaft, wie die Viskosität, herangezogen werden;

f. Speichern der Menge und der Zusammensetzung des aus dem System entnommenen Fluids, um die neue Zusammensetzung festzulegen, die es im Zeitalter $t_{i+1}$ hat;

g. die neue Zusammensetzung des Systems wird jene, die für die geochemische Modellierung beim Übergang in das folgende Zeitalter $(t_{i+1} \rightarrow t_{i+2})$ berücksichtigt wird.

7. Verfahren nach Anspruch 6, bei dem die durch geochemische Modellierung erhaltene Volumenvariation folgendermaßen geschrieben wird:

$\delta_{i+1} = \{(V_m)_{i+1} + (V_f)_{i+1}\} - \{(V_m)_i + (V_f)_i]\}$, wobei $(V_f)_i$ und $(V_m)_i$ das Fluidvolumen bzw. das Mineralienvolumen für das Zeitalter $t_i$ darstellen; und die Volumenvariation, die durch Beckenmodellierung erhalten wird, folgendermaßen geschrieben wird:
$\Delta_{i+1} = V_{i+1} - V_i$, wobei $V_i$ das durch Modellierung erhaltene Maschenvolumen darstellt.

8. Verfahren nach einem der vorhergehenden Ansprüche, bei dem die Fluide durch einen hohen Gehalt an Wasser und/oder Kohlendioxid und/oder Kohlenwasserstoffgas und/oder Wasserstoff und/oder Stickstoff und/oder Schwefelwasserstoff gekennzeichnet sind.

**Claims**

1. A computer-implemented method of predicting an amount and a composition of fluids of mineral origin generated in a sedimentary basin by reactions occurring among sedimentary rock minerals as said rocks are buried during the geological history of said basin, comprising:

i. acquiring geological data characteristic of said basin,
ii. constructing a representation of said basin by a grid,
iii. calculating, for at least one set of cells of said grid, by means of a basin model and of said geological data, the evolution of the following parameters: a depth of burial (z), a temperature (T), a pore pressure (P), a volume (V) and a porosity ($\Phi$) at successive ages $t_i$ representative of the geological history of the basin,
iv. determining, in each cell of said set of cells, a mineralogical or chemical rock composition from said geological data of the basin,
v. determining an amount and a composition of fluids of mineral origin within said set of cells, using a geochemical model and from said parameters and said mineralogical or chemical rock composition, and a thermodynamic database.

2. A method as claimed in claim 1 wherein, after stage v, an amount of said fluids that have migrated to reservoir rocks is determined by determining thermodynamic properties of said fluids, such as the distribution in various fluid or solid phases and/or the densities and viscosities of said phases, by means of an equation of state and of a migration model depending on said fluid properties.

3. A method as claimed in any one of the previous claims, wherein said set of cells corresponds to source cells, a source cell being a cell wherein the rock composition, the pressure and the temperature favour the formation of fluids of mineral origin.

4. A method as claimed in claim 3, wherein the temperature is above 250°C and the pressure is above 100 MPa.

5. A method as claimed in any one of the previous claims wherein, in stage v, the following stages are carried out:

- determining a sequence of reactions which, along a given Temperature-Pressure path, causes a rock of given chemical composition to go through a succession of stable mineral compositions,
- determining variations in the amounts of minerals and in the amounts of fluid constituents exchanged during said sequence of reactions.

6. A method as claimed in claim 5, wherein variations in the amounts of minerals and in the amounts of fluid constituents exchanged during this sequence of reactions are determined by carrying out the following stages:

a. identifying a stable system for the age $t_{i+1}$ from the composition at an age $t_i$,
b. identifying mineral reactions causing transition from the stable system for the age $t_i$ to the stable system for the age $t_{i+1}$,
c. carrying out a calculation of the quantitative balance, by mass and/or in number of moles, of the exchanges operated by these reactions,
d. carrying out a calculation of the quantitative balance, by volume, of these exchanges:

i. from a thermodynamic database for said minerals,
ii. from an equation of state allowing the composition and the density of each phase of the fluid to be calculated,

e. comparing volume variations obtained by geochemical modelling $\delta_{i+1}$ and by basin modelling $\Delta_{i+1}$ respectively: if $\delta_{i+1}$ exceeds $\Delta_{i+1}$ by an amount considered tolerable with regard to the expected precision of the fluid balances in the basin model, such as 1 % in relative value for example, the composition of the system is modified by removing a volume $\delta_{i+1} - \Delta_{i+1}$ of fluid, either according to the composition of the total fluid, or that of the least dense phase, or according to that of the least dense phase, or according to that of a mixture of each phase in proportions determined according to the values taken by a property calculated for the fluid, such as viscosity,
f. storing the amount and the composition of the fluid subtracted from the system so as to set the new composition it takes at the age $t_{i+1}$,
g. the new composition of the system becomes the composition taken into account for geochemical modelling upon transition to the next age $(t_{i+1} \to t_{i+2})$.

7. A method as claimed in claim 6, wherein the volume variation obtained by geochemical modelling is written as follows:

$$\delta_{i+1} = \left\{ (V_m)_{i+1} + (V_f)_{i+1} \right\} - \left\{ (V_m)_i + (V_f)_i \right\},$$

where $(V_f)_i$ and $(V_m)_i$ respectively represent the volume of fluid and the volume of minerals for the age $t_i$, and the volume variation obtained by basin modelling is written as follows:

$$\Delta_{i+1} = V_{i+1} - V_i,$$

where $V_i$ represents the cell volume obtained by modelling.

8. A method as claimed in any one of the previous claims, wherein the fluids are **characterized by** high water and/or carbon dioxide and/or hydrocarbon gas and/or hydrogen and/or nitrogen and/or hydrogen sulfide contents.

**FIG.1**

FIG. 2a

FIG. 2b

FIG.3

FIG.4

FIG.5

**FIG.6a**

**FIG.6b**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **L.M. CATHLES ; M. SCHOELL.** Modeling CO2 generation, migration, and titration in sedimentary basins. *Geofluids,* 2007, vol. 7, 441-450
- **P. ESKOLA.** The minerai facies of rocks. *Norsk Geologisk Tidsskrift,* 1921, vol. 6, 142-194
- **N.L. BOWEN.** Prograde metamorphism of siliceous limestone and dolomite. *Journal of Geology,* 1940, vol. 48, 225-274
- Integrated basin modeling helps to decipher petroleum systems. **J.L. RUDKIEWICZ et al.** Petroleum systems of South Atlantic Margin'', AAPG Memoir. 2000, vol. 73, 27-40
- **Z. DUAN ; N. MOLLER ; J.H. WEARE.** An equation of state (EOS) for CH4-CO2-H2O system: I. Pure systems from 0 to 1000 °C and 0 to 8000 bar. *Geochimica Cosmochimica Acta,* 1992, vol. 56, 2605-2617
- **R.G. BERMAN.** Internally-consistent thermodynamic data for minerais in the system Na2O-K2O-CaO-MgO-FeO-Fe2O3-Al2O3-SiO2-TiO2-H2O-CO. *Journal of Petrology,* 1988, vol. 29, 485-522
- **O. VIDAL ; T. PARRA ; F. TROTET.** A thermodynamic model for Fe-Mg aluminous chlorite using data from phase equilibrium experiments and natural pelitic assemblages in the 100°-600°C, 1-25 kb range. *American Journal of Science,* 2001, vol. 301, 557-592
- **T. PARRA ; O. VIDAL ; P. AGARD.** A thermodynamic model for Fe-Mg dioctahedral K-white micas using data from phase equilibrium experiments and natural pelitic assemblages. *Contribution to Mineralogy and Petrology,* 2002, vol. 143, 706-732
- **O. VIDAL ; T. PARRA ; P. VIEILLARD.** Thermodynamic properties of the Tschermak solid solution in Fe-chlorites: Application to natural examples and possible role of oxidation. *American Mineralogist,* 2005, vol. 90, 359-370
- **O. VIDAL ; V. DE ANDRADE ; E. LEWIN ; M. MUNOZ ; T. PARRA ; S. PASCARELLI.** P-T-deformation-Fe3+/Fe2+ mapping at the thin section scale and comparison with XANES mapping. Application to a garnet-bearing metapelite from the Sambagawa metamorphic belt (Japan). *Journal of Metamorphic Geology,* 2006, vol. 24, 669-683
- **T.J.B. HOLLAND ; R. POWELL.** An improved and extended internally consistent thermodynamic dataset for phases of petrological interest, involving a new equation of state for solids. *Journal of Metamorphic Geology,* 2011, vol. 29, 333-383
- **C. DE CAPITANI ; T.H. BROWN.** The computation of chemical equilibrium in complex systems containing non-ideal solutions. *Geochimica Cosmochimica Acta,* 1987, vol. 51, 2639-2652
- **C. DE CAPITANI ; K. PETRAKAKIS K.** The computation of equilibrium assemblage diagrams with Theriak/Domino software. *American Mineralogist,* 2010, vol. 95, 1006-1016
- **J.A.D. CONNOLLY.** Computation of phase equilibria by linear programming: A tool for geodynamic modeling and its application to subduction zone decarbonation. *Earth & Planetary Science Letters,* 2005, vol. 236, 524-541
- **R. POWELL ; T.J.B. HOLLAND ; B. WORLEY.** Calculating phase diagrams involving solid solutions via non-linear equations, with examples using THERMOCALC. *Journal of Metamorphic Geology,* 1998, vol. 16, 577-588
- **Z. DUAN, N. MOLLER & J.H. WEARE.** An equation of state (EOS) for CH4-CO2-H2O system: I. Pure systems from 0 to 1000°C and 0 to 8000 bar. *Geochimica Cosmochimica Acta,* 1992, vol. 56, 2605-2617
- **Z. DUAN, N. MOLLER & J.H. WEARE.** An equation of state (EOS) for CH4-CO2-H2O system: II. Mixtures from 50 to 1000 °C and 0 to 1000 bar. *Geochimica Cosmochimica Acta,* 1992, vol. 56, 2619-2631